# EUROPEAN PATENT APPLICATION

(11) **EP 2 213 249 A1**
(43) Date of publication of application: **04.08.2010**
(21) Application number: 10250139.2
(22) Date of filing: 28.01.2010
(51) Int. Cl.: A61B 17/06, A61B 17/00, A61B 17/02, A61B 17/34

(54) **Suture management system for surgical portal apparatus including springs**

(30) Priority: 29.01.2009 US 148139 P; 14.12.2009 US 636813
(71) Applicant: Tyco Healthcare Group LP, North Haven, CT 06473 (US)
(72) Inventor: Fowler, David N., Cheshire, CT 06410 (US)
(74) Representative: Soames, Candida Jane

(57) **Abstract**

A surgical portal apparatus for use during a surgical procedure involving at least one suture is provided. The surgical portal apparatus includes a portal member defining a longitudinal axis and having a longitudinal opening therethrough for receiving a surgical object, and a suture management device associated with the portal member. The suture management device includes a base segment and at least one spring element mounted to the base segment. The at least one spring is configured to selectively retain at least one suture in predetermined relation relative to the portal member.

## Description

### CROSS REFERENCE TO RELATED APPLICATION

The present application claims the benefit of and priority to U.S. Provisional Application Serial No. 61/148,139 filed on January 29, 2009, the entire contents of which are incorporated herein by reference.

### BACKGROUND

### Technical Field

The present disclosure relates to trocars and other surgical portal apparatus, and more particularly, relates to surgical portal apparatus including a suture management system that includes a spring.

### Background of Related Art

Trocars and other surgical portal apparatus are known, as are myriad procedures that may be preformed using such assemblies. Many of the minimally invasive procedures performed through access assemblies necessitate or are simplified by the use of one or more sutures passing through the surgical portal apparatus. Sutures extending into a body cavity through a surgical portal apparatus may be used to, for example, temporarily retain tissue, manipulate tissue, anchor tissue or operate peripheral devices. In an attempt to reduce the number of incision sites required to complete a given surgical procedure, a single surgical portal apparatus may be used to pass one or more sutures into a body cavity, in addition to providing access for one or more devices. A single anchor device may have numerous suture ends that extend therefrom and through the surgical portal apparatus. The sutures extending through the surgical portal apparatus may become tangled as each is manipulated or as one or more instruments are inserted and withdrawn from the assembly. Also, a surgeon may confuse the suture ends during the course of a surgery. Tangling or confusion of the suture ends may unnecessarily complicate the procedure and increase time necessary to complete the procedure.

Therefore, it would be beneficial to have a surgical portal apparatus that includes a suture management system.

### SUMMARY

Accordingly, a surgical portal apparatus for use during a surgical procedure involving at least one suture is provided. The surgical portal apparatus includes a portal member defining a longitudinal axis and having a longitudinal opening therethrough for receiving a surgical object, and a suture management device associated with the portal member. The suture management device includes a base segment and at least one spring element mounted to the base segment. The at least one spring is configured to selectively retain at least one suture in predetermined relation relative to the portal member. The base segment may include markings to identify the at least one spring element. The at least one spring element may include a coating to facilitate engagement with the at least one suture. The at least one spring element may be a coil spring. The coil spring may include a plurality of coil segments, whereby the at least one suture is selectively secured within adjacent coil segments. A plurality of coil springs may be radially spaced about the base segment.

In one embodiment, the base segment of the suture management device may be selectively releasable from the portal member. In the alternative, the base segment of the suture management device may be monolithically formed with the portal member. The base segment may define an opening in alignment with the longitudinal opening of the portal member. The portal member may include a housing and a sleeve extending from the housing.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
**FIG.1** 1 is a perspective side view of a surgical portal apparatus according to an embodiment of the present disclosure;
**FIG. 2** is a top view of the surgical portal apparatus of **FIC.1;**
**FIG. 3A** and **3B** are perspective views of the surgical portal apparatus of **FIGS. 1** and **2** as a suture "S" engages **(****FIG. 3A****)** and disengages **(****FIG. 3B****)** the surgical portal apparatus; and,
**FIG. 4** is a perspective side view of a suture management system according to another embodiment of the present disclosure.

### DETAILED DESCRIPTION

Referring now to the drawings wherein like reference numerals illustrate similar components throughout the several views, there is illustrated surgical portal apparatus **100** in accordance with the principles of the present disclosure. As shown in the drawings and as described throughout the following description, as is traditional when referring to relative positioning on an object, the term "proximal" refers to the end of the apparatus which is closer to the user and the term "distal" refers to the end of the apparatus which is further from the user. The surgical portal apparatus herein disclosed may be configured for use in various surgical procedures, including laparoscopic, endoscopic, arthroscopic and orthopedic surgery.

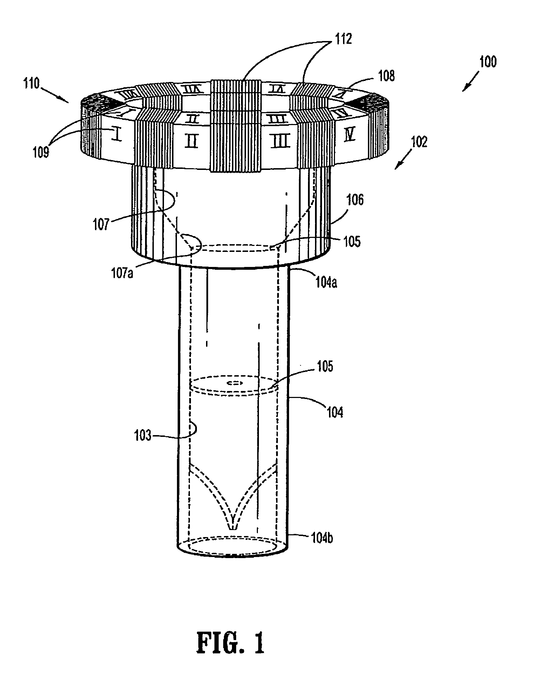

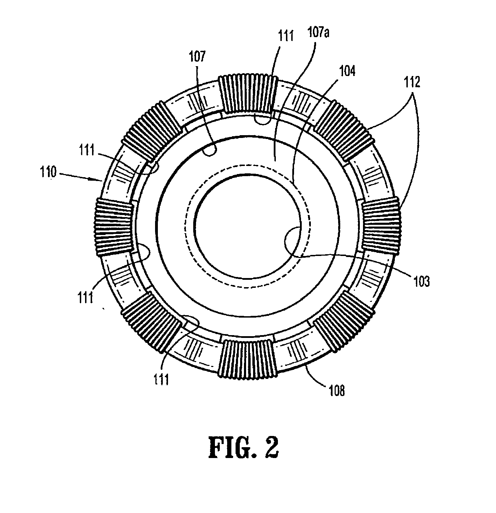

Referring initially to **FIGS. 1** and **2****,** an embodiment of a surgical portal apparatus of the present disclosure is shown generally as surgical portal apparatus or surgical portal device **100.** Surgical portal apparatus **100** includes a portal member **102** and a sleeve **104** extending distally from portal member **102.** Surgical portal apparatus **100** may be configured for use with any known endoscopic, laparoscopic, arthroscopic, orthopedic or other suitable instrument. Sleeve **104** is configured to be inserted through the skin of a patient and into a body cavity with the aid of an obturator (not shown). Sleeve **104** may instead include a blade or piercing tip for penetrating through the skin and into the body cavity. Sleeve **104** may be integral formed with portal member **102.** Alternatively, sleeve **104** may be configured for selectable engagement with portal member **102.**

Sleeve **104** forms a substantially tubular member having proximal and distal ends **104a, 104b** and defining a longitudinal passage **103** extending therebetween. Sleeve **104** may be composed of plastic, metal, polymers or the like. Sleeve **104** may be disposable, or in the alternative, reusable. Sleeve **104** may be rigid, or alternatively, sleeve **104** may be flexible. Sleeve **104** may be open, or instead, may be configured to include one or more seal members **105,** having any seal arrangement, for sealed reception of an instrument (not shown). Sleeve **104** may be of any configuration and of any length or diameter. Thus, it is appreciated that the embodiments of the present disclosure are not limited by the configuration of sleeve **104** and may be configured for use with any conceivable surgical portal apparatus configuration.

Still referring to **FIGS. 1** and **2****,** portal member **102** includes a substantially cylindrical housing **106** and a suture management system **110** operably connected to housing **106.** Housing **106** may be composed of plastic, metal polymers or the like. As discussed above, housing **106** is in operable connection with sleeve **104.** Housing **106** defines a longitudinal passage **107** and includes a tapered portion **107a** for directing one or more instruments (not shown) into passage **103** of sleeve **104.** Housing **106** may include one or more seal members **105b** having any seal arrangement. Housing **106** may further include an insufflation valve or port (not shown) configured to fill the body cavity of a patient with insufflation gas, saline or other suitable fluid.

With reference still to **FIGS. 1** and **2****,** surgical portal apparatus **100** further includes a suture management system **110.** Suture management system **110** includes a base segment **108** operably connected to a proximal end of housing **106.** As shown, base segment **108** is integrally formed with housing **106.** Alternatively, base segment **108** may be securely affixed to housing **106** with adhesives, welding or other suitable methods, or instead, base segment **108** may be selectively secured to housing **106** through friction fit, threading, mechanical fasteners or other suitable means. A series of arcuate slots **111** are formed between base segment **108** and housing **106.** Each of arcuate slots **111** are configured to permit a spring element **112** to be received about base segment **108.**

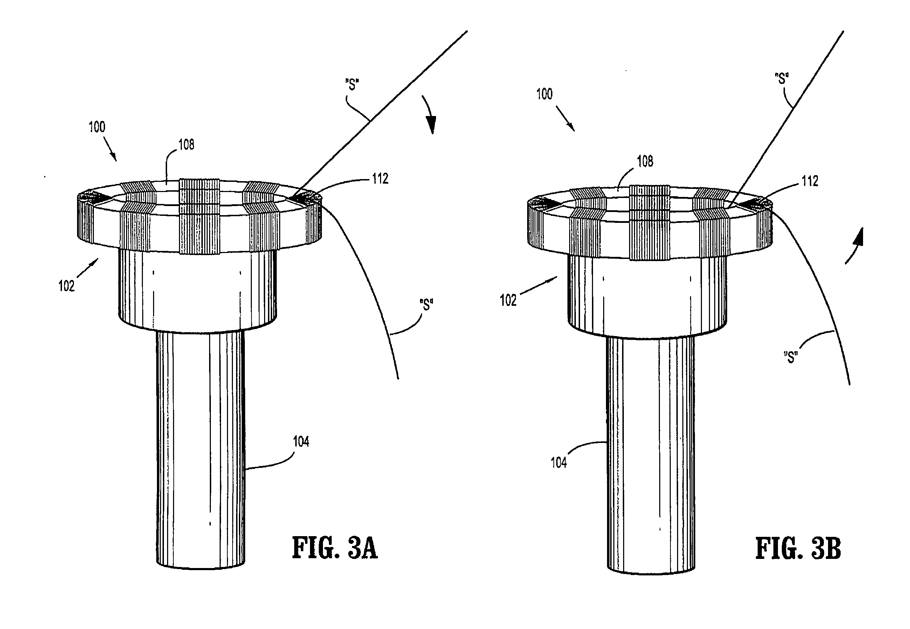

Still referring to **FIGS. 1** and **2****,** spring element **112** may include a coil spring having a plurality of coil segments. Alternatively, a single spring (not shown) may extend partially or entirely around base segment **108.** Springs **112** are configured to engage and retain one or more sutures "S" (**FIGS. 3A** and **3B**) extending through surgical portal apparatus **100.** Springs **112** may be composed of plastic, metal, polymer or other suitable material. Depending on the diameter of the sutures "S", spring **112** may require a tighter or looser coils to ensure secure engagement of suture "S" with spring **112.** Springs **112** may be coated (not shown) with rubber, polymer or other like material to increase the frictional engagement with suture "S" and/or to prevent damage to suture "S". A color coating (also not shown) may be added to springs **112** to assist in differentiating between the various sutures "S" retained therein. Alternatively, base segment **108** may include reference numerals or other markings **109** to differentiate springs **112** and sutures "S" secured therein. Markings **109** may be letters, numbers, symbols, colors or other identifying feature.

With reference now to **FIGS. 3A** and **3B****,** in operation, surgical portal apparatus 100 functions similar to conventional access assemblies. As discussed above, sleeve **104** may be inserted into a body cavity through an incision with the aid of an obturator (not shown), or alternatively, sleeve **104** may be fitted with a blade to create the incision. Once surgical portal apparatus **100** is properly received within the body cavity, the body cavity may be filled with insufflation gas, saline or other suitable fluid to permit greater access to the tissue therein. Surgical portal apparatus **100** is then ready to receive one or more sutures "S" and/or one or more endoscopic, laparoscopic, arthroscopic, orthopedic or other suitable device (not shown).

Referring initially to **FIG. 3A****,** suture "S" extends through surgical portal apparatus 100 and is pulled taut against one of springs **112.** By pulling suture "S" taut against spring **112,** a portion of suture "**S**" is forced between adjacent coils of spring **112.** Engagement of suture "S" within the coils of spring **112** locks suture "S" in place against base segment **108.** One or more sutures "S" may be secured in this manner utilizing the same or alternate springs **112.** At any time during the procedure, an instrument (not shown) may be inserted through passage **107 (****FIGS. 1** and **2****)** of surgical portal apparatus **100** without interfering with or tangling sutures "S". Turning now to **FIG. 3B****,** suture "S" may be released from between the coils of spring **112** by lifting suture "S" away from base segment **108.** Suture "S" may be re-secured to base segment **108** in the manner described above.

The use of suture management system **110** enables a clinician to actively organize and maintain one or more sutures "S" during a surgical procedure. Suture management system **110** reduces tangling of sutures "S" and prevents a clinician from becoming confused as to which suture "S" is which. Suture management system **110** further acts as a third hand for a clinician, maintaining tension on suture "S" extending from the body cavity. In this manner, suture "S" may be used to assist in retracting tissue or otherwise manipulating the tissue within the body cavity.

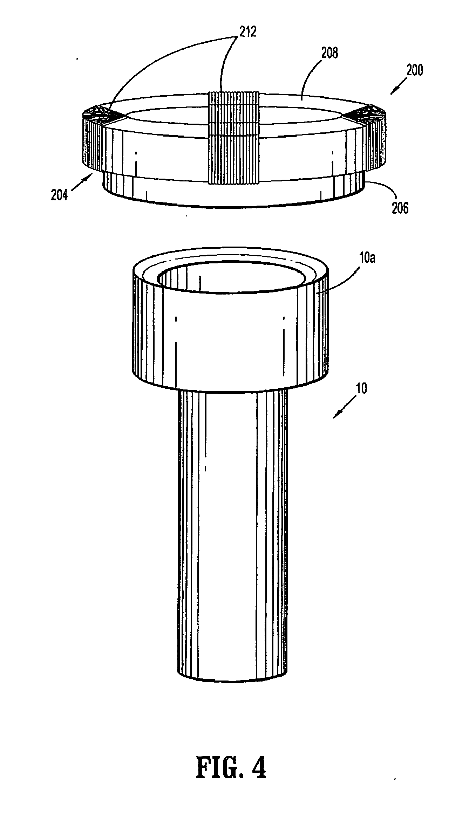

With reference now to **FIG. 4****,** an embodiment of a suture management system configured for use with a surgical portal apparatus is shown generally as suture management system **200.** Suture management system **200** defines a substantially annular portal member **204** including a housing **206** and a base segment **208** extending about a proximal end of housing **206.** Base segment **208** is substantially similar in form and function to base segment **108** described hereinabove. Base segment **208** includes a plurality of springs **212** spaced thereabout for engaging one or more sutures "S" **(****FIGS. 3A** and **3B**). As shown, housing **206** is configured to be selectively received on a proximal end **10a** of a surgical portal apparatus **10.** Housing **206** may be configured for use with a conventional surgical portal apparatus. Housing 206 may be frictionally fit, threaded, mechanically fastened, or otherwise secured to surgical portal apparatus **10.** Suture management system **210** is configured to operate as surgical portal apparatus **10,** with the added capability of managing multiple sutures "S" passing therethrough.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure.

## Claims

1. A surgical portal apparatus for use during a surgical procedure involving at least one suture, which comprises:
a portal member defining a longitudinal axis and having a longitudinal opening therethrough for receiving a surgical object; and
a suture management device associated with the portal member, wherein the suture management device includes a base segment and at least one spring element mounted to the base segment, the at least one spring being configured to selectively retain at least one suture in predetermined relation relative to the portal member.

2. The surgical portal apparatus of claim 1, wherein the base segment includes markings to identify the at least one spring element.

3. The surgical portal apparatus of claim 1 or claim 2, wherein the at least one spring element includes a coating to facilitate engagement with the at least one suture.

4. The surgical portal apparatus of any preceding claim, wherein the at least one spring element includes a coil spring.

5. The surgical portal apparatus of claim 4, wherein the coil spring includes a plurality of coil segments, whereby the at least one suture is selectively secured within adjacent coil segments.

6. The surgical portal apparatus of any preceding claim, including a plurality of coil springs radially spaced about the base segment.

7. The surgical portal apparatus of any preceding claim, wherein the base segment of the suture management device is selectively releaseable from the portal member.

8. The surgical portal apparatus of any preceding claim, wherein the base segment defines an opening in alignment with the longitudinal opening of the portal member.

9. The surgical portal apparatus of any preceding claim, wherein the portal member includes a housing and a sleeve extending from the housing.

10. The surgical portal apparatus of any preceding claim, wherein the base segment of the suture management device is monolithically formed with the portal member.

11. A suture management device for use with a portal member, the device comprising:
a base segment configured for operative engagement with a portal member; and
at least one spring element mounted to the base segment, the at least one spring being configured to selectively retain at least one suture in predetermined relation relative to the portal member.
